# EUROPEAN PATENT SPECIFICATION

(11) **EP 0 776 907 B1**
(45) Date of publication and mention of the grant of the patent: **08.03.2000**
(21) Application number: 96308341.5
(22) Date of filing: 15.11.1996
(51) Int. Cl.: C07K 14/52, C07K 1/22, G01N 30/48

(54) **Process for producing platelet factor-4**
Verfahren zur Herstellung von Plättchen-Faktor-4
Procédé de production du platelet facteur-4

(30) Priority: 17.11.1995 JP 32403595
(43) Date of publication of application: 04.06.1997
(73) Proprietor: JCR PHARMACEUTICALS Co., LTD., Ashiya, Hyogo 659 (JP)
(72) Inventor: Komurasaki, Yoshikazu, Hyogo (JP); Hirose, Takashi, Moriguchi, Osaka 570 (JP); Shindoh, Chihiro, Kobe, Hyogo (JP); Koh, Keihide, Kobe-, Hyogo (JP); Nishimuro, Satoshi, Higashi-Nada-ku, Kobe, Hyogo (JP)
(74) Representative: McMunn, Watson Palmer

(56) References cited:
- EP-A- 0 260 918
- EP-A- 0 589 719
- US-A- 5 258 502
- PROTEIN EXPRESSION AND PURIFICATION, vol. 2, 1991, pages 136-143, XP000652643 MYERS J.A. ET AL.: "Expression and Purification of Active Recombinant Platelet Factor 4 from Cleavable Fusion Protein"

## Description

The present invention relates to a process for producing platelet factor-4, such as by purification from blood, and an apparatus for use with the process.

In open intracardiac surgery, such as cardiac bypass surgery or arrest, intervention of bloodflow into and out of the heart is necessary for a limited period of time, and a heart-lung machine is used to take over temporarily the functions of the heart and the lungs. In order to prevent blood coagulation within the heart-lung machine, heparin is added to the blood flowing therein, and after the surgical operation protamine sulfate is administered to the patients to secure the recovery of their blood coagulation functions. In blood dialysis therapy for patients with renal function failures, heparin and protamine sulfate are used for a similar purpose. However, administration of protamine sulfate is known to cause a variety of side effects, such as allergic reaction, decrease in counts of platelets and white blood cells, hypotension, bradycardia, dyspnea, heat, transient rubeosis, nausea/vomiting and pulmonary edema (Japanese Pharmacopoeia, 12th revised edition; Viera, J., et al., J. 0. Amer. Surgeon, 50, 151, 1984).

On the other hand, it has been reported that a heparin-neutralizing substance appears to be present in platelets on the basis of variability in the heparin-neutralizing activity of platelets as measured by the magnitude of platelet counts (Conley, C. L., et al.. Proc. Soc. Exp. Biol. Med., 69, 284-287, 1948). Heparin-neutralizing substances in platelets have been reported as platelet factor-4 (hereinafter referred to briefly as "PF4"), β-thromboglobulin, low-affinity PF4, basic platelet protein, fibronectin, thrombospondin, etc., but it was discovered that PF4, among others, has the most potent affinity toward heparin. PF4 is a protein having a molecular weight of 7.8 kDa, and was reported to have heparin-neutralizing activity without accompaniment of the side effects (such as leukopenia, hypotension, pulmonary edema) produced by protamine sulfate (Cook, J. J., et al., Circulation, 85, 3, 1992). Such being the case, PF4 is expected to act as a heparin-neutralizing agent as a replacement for protamine sulfate.

In addition to the heparin-neutralizing effect, angiogenesis inhibitory activity can furthermore be expected of PF4. Angiogenesis, which is defined as the formation of new·capillaries, can be observed in healthy individuals on unusual occasions and in the ovaries or uteri according to the particular stage of the menstrual cycle. Its development is in many instances disadvantageous to living bodies except in the process of wound healing, such as with severe diabetic retinitis, retinopathy of prematurity, psoriasis vulgaris, and malignant tumors. Malignant tumor cells in particular are entirely free from contact inhibition and need the supply of oxygen and nutrients for their growth and they therefore vigorously induce angiogenesis to secure a route of supply for such nutrients and oxygen. Consequently, suppression of angiogenesis is considered to be effective for the suppression of malignant tumors. PF4 has been reported to elicit angiogenesis inhibitory activity (Maione, T. E., et al., Science, 247, 77-79, 1990). Furthermore the published results of a study in animals indicated that PF4 was effective for the suppression of malignant tumor cells (Shape, R. J., et al., J. Natl. Cancer Inst., 82, 848-853, 1990).

Heretofore, the purification of human PF4 has been carried out by means of heparin-immobilized affinity column chromatography using as a starting material a solution containing proteins released from platelets through stimulation of proteins such as thrombin, or a platelet extraction solution which is produced by allowing platelets to undergo hemolysis through freezing and thawing, then undergoing hypotonic treatment. (Levine, S. P., J. Biol. Chem., 251, 2, 324-328, 1976). However, these processes have drawbacks, such as the use of an expensive heparin-immobilized affinity column and the complicated procedures involved. As such they have not yet been established as an industrial process for the large-scale production of PF4 at reduced costs. Under these circumstances, the present inventors sought a novel PF4 production process which is suitable for large scale, mass production.

In Protein Expression and Purification 2, 136-143 (1991), recombinant PF4 is expressed as a fusion protein which has been modified to a highly charged fusion protein in such a way that allows improved purification without affecting the level of expression. The fusion protein is first separated from impurities using a S-Sepharose column and thereafter (in an additional step) fusion protein is cleaved (by CNBr cleavage) to recombinant platelet factor-4.

US-A-5,258,502 discloses a general process for purification of recombinant fusion proteins (platelet factor-4 is not mentioned). The fusion protein incorporates as a modification, a chitin binding portion which binds to chitin (as an adsorbent). After isolation, the chitin binding portion is then cleaved (in an additional step) to give the protein of interest.

Accordingly it is an object of the invention to provide a process for the production of PF4 which can be operated on industrial scale.

It is a further object of the invention to provide an improved process for the production of PF4.

The present inventors found that a PF4 containing solution, such as a platelet extraction solution, can be contacted with a sulfated chitin to thereby allow specific adsorption and bonding of PF4 onto the sulfated chitin, and that adsorbed PF4 can be eluted such as by increasing the salt concentration.

Thus, the present invention provides a process for producing platelet factor-4, characterized in that said process comprises contacting a solution containing platelet factor-4 with a sulfated chitin to adsorb the said factor, followed by elution of the said factor from the adsorption body.

A preferred embodiment of the invention comprises a process for purifying platelet factor-4 from human blood comprising centrifuging a volume of human blood to separate out the plasma, mixing the remaining blood cells with salt solution and then centrifuging further to remove the white blood cells, thereafter treating the separated supernatant to recover platelet factor-4, admixing the resulting platelet extraction solution with a salt, contacting with a sulfated chitin to adsorb said factor-4, then eluting the factor-4 with a salt solution which is more concentrated than the factor-4 solution which contacted the sulfated chitin.

A starting material of a solution containing platelet factor-4 which is usable in the present invention may be, for example, platelet extraction solutions produced by allowing platelets as recovered from human blood by centrifugation to undergo freezing and thawing, hypotonic treatment or homogenization for fine pulverization, and solutions containing PF4 which is released from platelets through stimulation with compounds such as thrombin.

Sulfated chitins are manufactured by sulfating a chitin produced from the outer shell of crustaceans.

Sulfated chitins may have the hydroxyl groups of chitin partly or entirely sulfated and may furthermore have the acetamino groups at the 2-position partially deacetylated or sulfated.

A PF4-containing solution is desirably contacted with a sulfated chitin while keeping the pH of the solution around neutral more specifically 6 to 9, preferably about pH 7.5. The concentration of a salt in the solution is desirably less than 1.5 M, preferably about 0.4 M. Examples of suitable salt include alkali metal salts such as sodium chloride and potassium chloride, although use may be made of other salts, such as sodium sulfate, unless they interfere with the adsorption of PF4.

The amount of PF4-containing solution which is admixed with a sulfated chitin is not particularly limited, but it is preferable to use 10 to 100 ml of a sulfated chitin against a platelet extraction solution of about 1 liter of human blood.

The length of time for which the PF4-containing solution is contacted with a sulfated chitin is not specifically restricted, and such contacting procedure can be carried out by employing either a column method or batch method. The contacting procedure is effected to thereby allow PF4 in the solution to be adsorbed and bond specifically onto the sulfated chitin.

PF4 as adsorbed onto a sulfated chitin can be eluted at a pH of about neutral, more specifically at a pH in the range of 6 to 9 inclusive, preferably about pH 7.5 using a solution having a higher salt concentration than the PF4-containing solution employed for adsorption. Preferably the salt concentration of the eluting solution is not less than 0.5 M, more preferably not less than 1 M. Preferred examples of such salt include alkaline metal salts such as sodium chloride and potassium chloride, but because the adsorbed PF4 is eluted by virtue of a change in ionic strength, other salts may be usable.

PF4 as obtained in the above manner can furthermore be desalted for purification by use of, for example, Sephacryl S-100 (produced by Pharmacia of Sweden), or TSK-GEL G2000 (produced by Tohsoh Inc. of Japan).

Further aspects of the invention relate to:
(a) an adsorption column for the purification of platelet factor-4 containing, as an adsorbent, a sulfated chitin; and
(b) the use of sulfated chitin as an adsorbent for platelet factor-4 in the purification of platelet factor-4.

The following non-limiting examples further illustrate the present invention in more detail, with reference to the attached drawings in which:
Figure 1 is a reproduction of Western blot analysis (photograph) for platelet factors-4 produced by the process of the present invention and the conventional process; and
Figure 2 is a graph illustrating concentration versus the heparin-neutralizing activity of platelet factor-4 produced by the process of the present invention and platelet factor-4 produced by a conventional process.

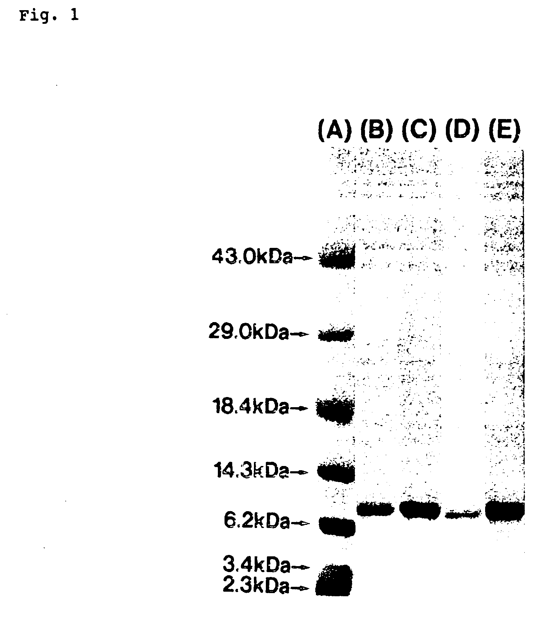

The nomenclature for Figure 1 is as follows:
- (A), (B) and (D):: SDS-PAGE
- (C) and (E) :: Western blot analysis
- (A) :: Molecular-weight marker
- (B) and (C) :: PF4 as obtained by the process of the present invention
- (D) and (E) :: PF4 as obtained by the conventional process

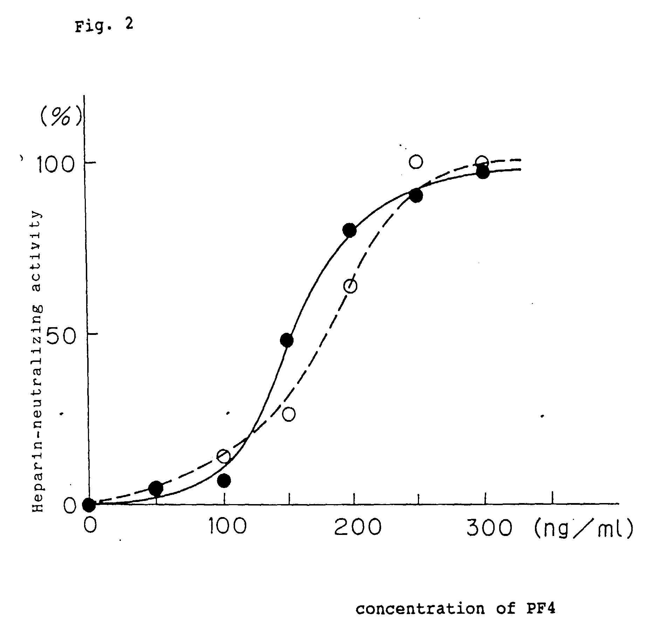

The nomenclature for Figure 2 is as follows:
1. The marks "●" and "○" designate PFs produced individually by the process of the present invention and the conventional process.
2. Heparin-neutralizing activity = A/B x 100, %
   Where:
   - A =: Initial rate of synthetic-substrate degradation by thrombin when admixed with PF4
   - B =: Initial rate of synthetic-substrate degradation by thrombin in the absence of heparin.

### Example 1

### Production of PF4 in accordance with the present invention:

Human blood drawn was centrifuged for 6 min at 22°C and at 3,500 x g to separate out the plasma, and the remaining blood cells were admixed with a 9-fold volume of a 0.155M ammonium chloride solution and left standing for 10 min at room temperature. Then, centrifugation was effected for 7 min. at 4°C and at 220 x g, and the white blood cells which precipitated were removed. The resultant supernatant after removal of the white blood cells was furthermore centrifuged for 10 min. at 4°C and at 1,200 x g, and the platelets which precipitated were recovered, followed by washing with isotonic saline and freezing at -20°C. The frozen platelets were thawed, admixed with a 4-fold volume of 15 mM Tris hydrochloride buffer (pH 7.6) and centrifuged for 10 min. at 4°C and at 700 x g, to provide, as separated supernatant, a platelet extraction solution. The platelet extraction solution was admixed with sodium chloride to the final concentration of 0.4 M and added to a sulfated chitin (supplied by Fuji Spinning Co. of Japan under the tradename of Sulfonated Chitopal), then equilibrated with 15 mm Tris hydrochloride/0.4M sodium chloride solution. The bonded PF4 was eluted with 15 mM Tris hydrochloride/1.0 M sodium chloride solution.

In accordance with the conventional process, on the other hand, the platelet extraction solution after being admixed with sodium chloride to a final concentration of 1.0 M, was added at a volume ratio of 1:1 to heparin-Sepharose (produced by Pharmacia Co. of Sweden) equilibrated with 15 mM Tris hydrochloric acid/1.0 M sodium chloride solution in the same volume as that of the above-mentioned sulfated chitin, and the bonded PF4 was eluted with a 2.0 M sodium chloride solution buffered with 15 mM Tris hydrochloric acid.

As a result, it was found that the process of the present invention produced 20 mg of PF4 per liter of human blood, whereas the conventional process yielded 10 mg of PF4 per liter of human blood.

### Test Example 1

### SDS-Polyacrylamide electrophoresis

PF4 as obtained in Example 1 and a dodecylsulfonating agent (composed of 2 % SDS, 10 M urea, 1 mM EDTA, 0. 1 M sucrose, 5 % β-mercaptoethanol and 10 mM Tris-HCl, pH 7.4) were mixed in equal volumes, and the mixture was heated for 5 min. at 100°C and then electrophoresed on a polyacrylamide gel (produced by Dai-Ichi Kagaku Yakuhin Co. of Japan) with a concentration gradient of 15 % to 25 % in the presence of 0.1 % SDS. The protein separated on the gel was stained with 0.25 % Coomassie Brilliant Blue R-250 (produced by Bio-Rad Co. of USA); dissolved in 50 % ethanol and 10 % acetic acid), giving rise to a single band at 7.8 kDa (see Figure 1) .

### Test Example 2

### Western blotting method:

A protein developed on a polyacrylamide gel in the same manner as described in Test Example 1 was transferred electrically (conducted by supplying an electric current of 200 mA for 30 min. with use of Tris-glycine buffer containing 20 % % ethanol) to a PVDF membrane (produced by Dai-Ichi Kagaku Yakuhin Co. of Japan) in a Salz Blot device (manufactured by Sartrius Co. of Germany), and the membrane was washed thoroughly with TBS (20 mM Tris and 500 mM NaCl, pH 7.5) and then held in TBS containing 50 % defatted milk for 1 hour to thereby block the non-adsorbed portion. Thereafter, the membrane was reacted with a 500-fold dilution of goat's anti-human-PF4 antiserum (produced by ATAB Co. of USA) with TTBS (0.05 % Tween 20, TBS) containing 1 % BSA for 1 hour at room temperature, then washed thoroughly with TTBS and reacted with a 500-fold dilution of anti-goat's IgG labelled with alkaline phosphatase (produced by BioMakor Co. of USA) with TTBS containing 1 % BSA. After conclusion of the reaction, coloring reaction with a substrate solution containing Nitro Blue Tetrazolium and bromochloroindolylic acid showed a single band in the same molecular weight region as that of SDS-polyacrylamide gel electrophoresis (see Figure 1).

### Test Example 3

### Amino acid sequencing

The PF4 as obtained in Example 1 was transferred onto a PVDF membrane by the above-described procedure, followed by washing with TBS and dying with 1 % acetic acid containing 0.1 % Bonsoh S. The membrane was then washed successively with deionized water and methanol, and dried. The dyed portion was cut and subjected to measurement of 9 N-terminal residues with an amino-acid sequence analysis device, with the result that it was in agreement with the sequence previously reported (Duel, T. F. et al., Proc. Natl. Acad. Sci. USA, 74, 6, 2256-2258, 1977).

### Test Example 4

### Heparin-neutralizing activity of PF4:

Antithrombin III inhibits the activity of thrombin by binding to it, whereby such inhibition is promoted remarkably under the presence of heparin. By adding PF4 to a solution admixed with antithrombin III, thrombin and heparin, the recovered activity of thrombin was assayed and determined on the basis of the initial rate of synthetic-substrate degradation by thrombin, followed by calculation of the heparin-neutralizing activity of PF4.

Heparin (final concentration of 7.2 x 10⁻³ units/ml; produced by Wako Pure Chemicals Ind., Ltd. of Japan), PF4 as obtained in Example 1 or produced by the conventional process, antithrombin III (final concentration of 2.9 µg/ml; produced by Seikagaku Kogyo Co. of Japan) and 40 mM HEPES (pH 7.4) were mixed to make the total volume of 2 ml, and the mixture was left on standing for 30 sec. at room temperature and admixed with 15 µl of thrombin (final concentration of 0.18 µg/ml; produced by Sigma Co. of USA), followed by standing for 1 min at room temperature. After addition of Coloring Substrate S-2238 (at a final concentration of 0.1 mM/ml (produced by Dai-Ichi Kagaku Co. of Japan), the degradation of the coloring substrate by thrombin was determined in a time-course manner by measurement of the absorbance at a wavelength of 405 nm.

The results indicated that the PF4 as obtained in accordance with the present invention exhibited specific activity equal to that of the PF4 produced by the conventional process (the Heparin-Sepharose method) (see Fig. 2).

## Claims

1. A process for producing platelet factor-4, characterized in that said process comprises contacting a solution containing platelet factor-4 with a sulfated chitin to adsorb the said factor-4, followed by elution of the said factor from the adsorption body.

2. A process as claimed in Claim 1, wherein the sulfated chitin is a chitin having only some of its hydroxyl groups sulfated.

3. A process as claimed in Claim 1, wherein the sulfated chitin has all of its hydroxyl groups sulfated.

4. A process as claimed in any one of the preceding claims, wherein the sulfated chitin has its acetamino groups intact.

5. A process as claimed in any one of Claims 1 to 3, wherein the sulfated chitin has its acetamino groups partially deacetylated and sulfated.

6. A process as claimed in any one of the preceding claims, wherein the solution containing platelet factor-4 to be contacted with the sulfated chitin contains a salt, and platelet factor-4 is eluted from the sulfated chitin
with a solution containing a salt in a higher concentration than in said platelet factor-4 solution.

7. A process as claimed in Claim 6, wherein the solution containing platelet factor-4 contains less than 1.5M of salt, and elution of platelet factor-4 is carried out from the sulfated chitin with a solution containing a higher concentration of a salt, with the said salt concentration being at least 0.5M.

8. A process as claimed in Claim 7, wherein concentration of salt in the contacting solution containing platelet factor-4 is about 0.4M, and the concentration of salt in the eluting solution is at least 1M.

9. A process as claimed in any one of the preceding claims wherein contact of platelet factor-4 with the sulfated chitin and elution of the said factor from the adsorption body are effected at a pH in the range of 6 to 9 inclusive.

10. A process as claimed in any one of the preceding claims wherein about 1 litre of the solution containing platelet factor-4 is added to 10 to 100ml inclusive of sulfated chitin.

11. A process as claimed in any one of the preceding claims wherein the solution containing platelet factor-4 which is contacted with the sulfated chitin, is derived from human blood.

12. A process as claimed in Claim 11 for purifying platelet factor-4 from human blood, said process comprising centrifuging a volume of human blood to separate out the plasma, mixing the remaining blood cells with a salt solution and then centrifuging further to remove the white blood cells, thereafter treating the separated supernatant to recover platelet factor-4, admixing the resulting platelet extraction solution with a salt, contacting with a sulfated chitin to adsorb said factor-4, then eluting the factor-4 with a salt solution which is more concentrated than the factor-4 solution which contacted the sulfated chitin.

13. The use of sulfated chitin as an adsorbent for platelet factor-4 in the purification of platelet factor-4.

14. The use as claimed in Claim 13 wherein the platelet factor-4 is derived from human blood.

15. An adsorption column for the purification of platelet factor-4 according to any one of the preceding claims containing, as an adsorbent, a sulfated chitin.

## Patentansprüche

1. Verfahren zur Herstellung des Plättchenfaktors 4, dadurch gekennzeichnet, daß das Verfahren das Kontaktieren einer Lösung mit einem Gehalt an Plättchenfaktor 4 mit einem sulfatierten Chitin zur Adsorption dieses Faktors 4 mit nachfolgender Elution des Faktors aus dem Adsorptionskörper umfaßt.

2. Verfahren nach Anspruch 1, bei dem das sulfatierte Chitin ein Chitin darstellt, bei dem nur einige seiner Hydroxylgruppen sulfatiert sind.

3. Verfahren nach Anspruch 1, bei dem das sulfatierte Chitin ein Chitin darstellt, dessen sämtliche Hydroxylgruppen sulfatiert sind.

4. Verfahren nach einem der vorstehenden Ansprüche, bei dem die Acetaminogruppen des sulfatierten Chitins intakt sind.

5. Verfahren nach einem der Ansprüche 1 bis 3, bei dem die Acetaminogruppen des sulfatierten Chitins teilweise desacety-liert und sulfatiert sind.

6. Verfahren nach einem der vorstehenden Ansprüche, bei dem die Lösung mit einem Gehalt an Plättchenfaktor 4, die mit dem sulfatierten Chitin in Kontakt gebracht werden soll, ein Salz enthält, und der Plättchenfaktor 4 mit einer Lösung mit einem Gehalt an einem Salz in einer höheren Konzentration im Vergleich zur Lösung des Plättchenfaktors 4 aus dem sulfatierten Chitin eluiert wird.

7. Verfahren nach Anspruch 6, bei dem die Lösung mit einem Gehalt an Plättchenfaktor 4 weniger als 1,5 M Salz enthält und die Elution des Plättchenfaktors 4 aus dem sulfatierten Chitin mit einer Lösung durchgeführt wird, die eine höhere Salzkonzentration enthält, wobei die Salzkonzentration mindestens 0,5 M beträgt.

8. Verfahren nach Anspruch 7, bei dem die Salzkonzentration in der in Kontakt zu bringenden Lösung mit einem Gehalt an Plättchenfaktor 4 etwa 0,4 M ist, wobei die Konzentration an Salz in der Elutionslösung mindestens 1 M beträgt.

9. Verfahren nach einem der vorstehenden Ansprüche, bei dem das Kontaktieren des Plättchenfaktors 4 mit dem sulfatierten Chitin und die Elution dieses Faktors aus dem Adsorptionskörper bei einem pH-Wert im Bereich von 6 bis 9 einschließlich durchgeführt werden.

10. Verfahren nach einem der vorstehenden Ansprüche, bei dem etwa 1 Liter der Lösung mit einem Gehalt an Plättchenfaktor 4 zu 10 bis 100 ml einschließlich sulfatierten Chitins hinzugegeben wird.

11. Verfahren nach einem der vorstehenden Ansprüche, bei dem die Lösung mit einem Gehalt an Plättchenfaktor 4, die mit dem sulfatierten Chitin in Kontakt gebracht wird, von Humanblut stammt.

12. Verfahren nach Anspruch 11 zur Reinigung von Plättchenfaktor 4 aus Humanblut, wobei das Verfahren das Zentrifugieren eines Volumens Humanblut zur Abtrennung des Plasmas, Vermischen der verbliebenen Blutzellen mit einer Salzlösung und das nachfolgende weitere Zentrifugieren zum Entfernen der weißen Blutzellen, anschließendes Behandeln des abgetrennten Überstandes zur Gewinnung des Plättchenfaktors 4, Vermischen der so gewonnenen Plättchenextraktionslösung mit einem Salz, Kontaktieren mit einem sulfatierten Chitin zur Adsorption dieses Faktors 4 und schließlich die Elution des Faktors 4 mit einer Salzlösung umfaßt, die eine höhere Konzentration auf weist als die Faktor-4-Lösung, welche mit dem sulfatierten Chitin in Kontakt gebracht worden war.

13. Verwendung von sulfatiertem Chitin als Adsorptionsmittel für den Plättchenfaktor 4 bei der Reinigung des Plättchenfaktors 4.

14. Verwendung nach Anspruch 13, bei dem der Plättchenfaktor 4 von Humanblut stammt.

15. Adsorptionssäule für die Reinigung des Plättchenfaktors 4 nach einem der vorstehenden Ansprüche mit einem Gehalt an einem sulfatierten Chitin als Adsorptionsmittel.

## Revendications

1. Procédé de production de facteur plaquettaire 4, caractérisé en ce que ledit procédé comprend la mise en contact d'une solution contenant le facteur plaquettaire 4 avec de la chitine sulfatée pour adsorber ledit facteur 4, puis l'élution dudit facteur du corps adsorbant.

2. Procédé selon la revendication 1, dans lequel la chitine sulfatée est une chitine dont un certain nombre seulement de groupes hydroxy sont sulfatés.

3. Procédé selon la revendication 1, dans lequel les groupes hydroxy de la chitine sont sulfatés en totalité.

4. Procédé selon l'une quelconque des revendications précédentes, dans lequel la chitine sulfatée présente des groupes acétamino intacts.

5. Procédé selon l'une quelconque des revendications 1 à 3, dans lequel les groupes acétamino de la chitine sulfatée sont en partie desacétylés et sulfatés.

6. Procédé selon l'une quelconque des revendications précédentes, dans lequel la solution contenant le facteur plaquettaire 4 devant être mise en contact avec la chitine sulfatée contient un sel, et le facteur plaquettaire 4 est élué de la chitine sulfatée à l'aide d'une solution contenant un sel en plus forte concentration que ladite solution de facteur plaquettaire 4.

7. Procédé selon la revendication 6, dans lequel la solution contenant le facteur plaquettaire 4 a une concentration en sel inférieure à 1,5 M, et l'élution du facteur plaquettaire 4 de la chitine sulfatée s'effectue au moyen d'une solution contenant une plus forte concentration en sel, ladite concentration en sel étant d'au moins 0,5 M.

8. Procédé selon la revendication 7, dans lequel la concentration en sel de la solution de contact contenant le facteur plaquettaire 4 est d'environ 0,4 M et la concentration en sel de la solution d'élution est d'au moins 1M.

9. Procédé selon l'une quelconque des revendications précédentes, dans lequel la mise en contact du facteur plaquettaire 4 avec la chitine sulfatée et l'élution dudit facteur du corps adsorbant s'effectuent à pH compris entre 6 et 9 inclus.

10. Procédé selon l'une quelconque des revendications précédentes, dans lequel environ 1 litre de la solution contenant du facteur plaquettaire 4 est ajouté à 10 à 100 ml inclus de chitine sulfatée.

11. Procédé selon l'une quelconque des revendications précédentes, dans lequel la solution contenant du facteur plaquettaire 4 qui est mise en contact avec la chitine sulfatée, dérive de sang humain.

12. Procédé selon la revendication 11 pour la purification du facteur plaquettaire 4 à partir de sang humain, ledit procédé comprenant la centrifugation d'un volume de sang humain en vue de séparer le plasma, le mélange des cellules sanguines restantes avec une solution salée et la poursuite de la centrifugation en vue d'éliminer les leucocytes, le traitement ensuite du surnageant séparé pour isoler le facteur plaquettaire 4, le mélange de la solution d'extraction plaquettaire résultante avec un sel, la mise en contact avec de la chitine sulfatée en vue d'adsorber ledit facteur 4, puis l'élution du facteur 4 avec une solution salée qui est plus concentrée que la solution de facteur 4 mise auparavant en contact avec la chitine sulfatée.

13. Utilisation de chitine sulfatée comme adsorbant pour le facteur plaquettaire 4 dans la purification du facteur plaquettaire 4.

14. Utilisation selon la revendication 13, dans laquelle le facteur plaquettaire 4 dérive de sang humain.

15. Colonne d'adsorption pour la purification du facteur plaquettaire 4 selon l'une quelconque des revendications précédentes contenant, comme adsorbant, de la chitine sulfatée.
